# EUROPÄISCHE PATENTSCHRIFT

(11) **EP 1 312 599 B1**
(45) Veröffentlichungstag und Bekanntmachung des Hinweises auf die Patenterteilung: **08.03.2006**
(21) Anmeldenummer: 02024433.1
(22) Anmeldetag: 29.10.2002
(51) Int. Cl.: C07C 209/86, C07C 211/08, C07D 211/12, C07D 207/04, C07D 265/30

(54) **Verfahren zur Auftrennung von wasserhaltigen Rohamingemischen aus der Aminsynthese**
Process for separating mixtures of crude, water-containing amines originating from the amine synthesis
Procédé de séparation d'un mélange d'amines bruts contenant de l'eau provenant de la synthèse de l'amine

(30) Priorität: 30.10.2001 DE 10153411
(43) Veröffentlichungstag der Anmeldung: 21.05.2003
(73) Patentinhaber: BASF Aktiengesellschaft, 67063 Ludwigshafen (DE)
(72) Erfinder: Wölfert, Andreas, Dr., 74906 Bad Rappenau (DE); Rütter, Heinz, Dr., 67126 Hochdorf-Assenheim (DE); Rittinger, Stefan, Dr., 25050 Kuantan, Pahang (MY); Wehinger, Mark, 68229 Mannheim (DE); Alemany, Aurelie, 68159 Mannheim (DE); Schmidt, Willi, 67069 Ludwigshafen (DE)
(74) Vertreter: Isenbruck, Günter

(56) Entgegenhaltungen:
- EP-A- 0 034 400
- EP-A- 0 881 211
- DE-A- 2 723 474
- DE-A- 2 902 302

## Beschreibung

Die Erfindung betrifft ein Verfahren zur Auftrennung eines Gemischs, das ein oder mehrere Amine, Wasser, Leichtsieder und gegebenenfalls Schwersieder enthält.

Bei der Umsetzung von Ammoniak, primären oder sekundären Aminen mit Alkoholen oder mit Aldehyden in Gegenwart von Wasserstoff entsteht als Reaktionsprodukt unter anderem Wasser, welches häufig mit dem gebildeten Produkt-Amin ein azeotrop siedendes Amin/Wasser-Gemisch bildet. Daneben liegen in dem Produktgemisch Leichtsieder mit niedrigerem Siedepunkt als dem des Amin/Wasser-Azeotrops, beispielsweise unumgesetzter Ammoniak oder Edukt-Amin, und Schwersieder mit höherem Siedepunkt als dem des Produkt-Amins, beispielsweise höhermolekulare Nebenprodukte, vor.

GB 1,102,370 beschreibt ein Verfahren der Extraktivdestillation eines Ethylendiamin/Wasser-Gemischs, bei dem in einer ersten Destillationskolonne das Ethylen/Wasser-Rohgemisch verdampft und der aufsteigende Dampf mit im Gegenstrom fließender wässriger Natronlauge in Kontakt gebracht wird. Am Kopf der ersten Kolonne wird ein wasserarmes Ethylendiamin/Wasser-Gemisch mit einer Aminkonzentration oberhalb des azeotropen Punktes erhalten, das in einer zweiten Kolonne durch einfache Rektifikation weiter aufdestilliert wird. Am Kopf der zweiten Kolonne wird reines Ethylendiamin erhalten, am Sumpf der zweiten Kolonne ein Ethylendiamin/Wasser-Gemisch, das mit dem Rohgemisch vereinigt und in die Extraktivdestillation zurückgeführt wird.

DE-A 29 02 302 beschreibt ein Verfahren zur Trennung von Ethylamin-Gemischen, bei dem ein Diethylamin, Triethylamin, Ethanol, Wasser und gegebenenfalls Monoethylamin enthaltendes Gemisch mit Wasser und einem mit Wasser nicht mischbaren Lösungsmittel extrahiert wird, wobei eine wässrige und eine mit Wasser nicht mischbare Phase erhalten werden. Die beiden Phasen werden getrennt und destillativ weiter aufgearbeitet. Als mit Wasser nicht mischbare Lösungsmittel werden n-Butan, n-Hexan und Cumol eingesetzt.

DE-A 27 23 474 beschreibt ein Verfahren zur Auftrennung eines Wasser, Mono-, Di- und Triethylamin enthaltenden Gemischs, bei dem durch Destillation ein im wesentlichen wasserfreies Gemisch aus Mono- und Diethylamin und Triethylamin abgetrennt und durch Destillation Monoethylamin von dem wasserfreien Gemisch aus Mono- und Diethylamin abgetrennt wird.

EP-A 0 881 211 beschreibt ein Verfahren zur Herstellung von wasserfreiem 2-Amino-1-methoxypropan, bei dem in einem Extraktionsschritt ein 2-Amino-1-methoxypropan enthaltendes wässriges Reaktionsgemisch mit Natronlauge unter Ausbildung einer Natronlauge enthaltenden wässrigen Phase und einer 1-Amino-1-methoxypropan enthaltenden Phase versetzt wird, die wässrige Phase abgetrennt und in einem Destillationsschritt die 2-Amino-1-methoxypropan enthaltende Phase destilliert wird, wobei zuerst ein Azeotrop aus Wasser und 2-Amino-1-methoxypropan gewonnen wird, das in den Extraktionsschritt zurückgeführt wird, und dann wasserfreies 2-Amino-1-methoxypropan gewonnen wird.

Aufgabe der Erfindung ist, ein verbessertes Verfahren zur Auftrennung von wasserhaltigen Rohamingemischen aus der Aminsynthese bereitzustellen, das für eine Vielzahl unterschiedlicher Rohamingemische geeignet ist.

Gelöst wird die Aufgabe durch ein Verfahren zur Auftrennung eines aminhaltigen Gemischs, das ein oder mehrere Amine, Wasser, Leichtsieder und gegebenenfalls Schwersieder enthält, mit den Schritten (i) bis (v):
(i) destillative Abtrennung einer ersten Leichtsiederfraktion von dem aminhaltigen Gemisch,
(ii) gegebenenfalls destillative Abtrennung einer (ersten) Schwersiederfraktion von dem aminhaltigen Gemisch,
(iii) Extraktion des aminhaltigen Gemischs mit Natronlauge unter Gewinnung einer wässrigen, Natronlauge enthaltenden ersten Phase und einer wässrig-organischen, Amin, weitere Leichtsieder und gegebenenfalls (weitere) Schwersieder enthaltenden zweiten Phase,
(iv) Destillation der wässrig-organischen zweiten Phase unter Gewinnung von im wesentlichen wasserfreiem Amin als Sumpfabzug oder Seitenabzug im Abtriebsteil der Destillationskolonne, einem Amin/Wasser-Azeotrop als Seitenabzug im Verstärkungsteil der Kolonne und einer weiteren Leichtsiederfraktion als Kopfabzug, und Rückführung des Amin/Wasser-Azeotrops in den Extraktionsschritt (iii),
(v) gegebenenfalls weitere Aufreinigung oder Auftrennung des im wesentlichen wasserfreien Amins.

Das Verfahren wird kontinuierlich durchgeführt. Durch die Entnahme einer Leichtsiederfraktion als Kopfabzug der Destillationskolonne in Schritt (iv) wird eine Anreicherung von Leichtsiedern durch die Rückführung von Amin/Wasser-Azeotrop in den Extraktionsschritt (üi) bei kontinuierlicher Betriebsweise vermieden. Unter "Amin" wird auch ein Gemisch aus mehreren Aminen verstanden.

Optional kann vor dem Extraktionsschritt (iii) eine Schwersiederabtrennung (ii) durchgeführt werden, bevorzugt wird sie durchgeführt. Überraschender Weise wird dadurch die unerwünschte Bildung von Feststoffen im Extraktionsschritt (iii) vermieden.

Der Schwersiederabtrennung (ii) kann eine erste Leichtsiederabtrennung (i) vorgeschaltet sein. Bevorzugt wird diese durchgeführt, insbesondere wenn bei der Aminsynthese mit einem Überschuss an Edukt-Amin gearbeitet wird.

Im wesentlichen wasserfreies Amin kann im Destillationsschritt (iv) als Sumpfabzug oder aber als Seitenabzug im Abtriebsteil der Destillationskolonne erhalten werden. Besonders bevorzugt ist eine Verfahrensvariante, bei der im wesentlichen wasserfreies Amin als Seitenabzug im Abtriebsteil der Kolonne gewonnen und weitere Schwersieder enthaltendes Amin als Sumpfabzug gewonnen werden, wobei dieser Sumpfabzug in die Schwersiederabtrennung (ii) zurückgeführt wird.

Das im Destillationsschritt (iv) anfallende, im wesentlichen wasserfreie Amin kann in einem nachgeschalteten Destillationsschritt (v) weiter aufgereinigt werden oder aufgetrennt werden. Ein solcher Destillationsschritt ist zur Gewinnung der reinen Amine erforderlich, wenn das Ausgangsgemisch zwei oder mehr Amine enthält, die mit Wasser Azeotrope mit stark ähnlichem Siedepunkt bilden. Beispiele hierfür sind Azeotrope, deren Siedepunkte sich um nicht mehr als 10 °C unterscheiden.

Nachstehend werden zwei Ausführungsformen der Erfindung näher erläutert.

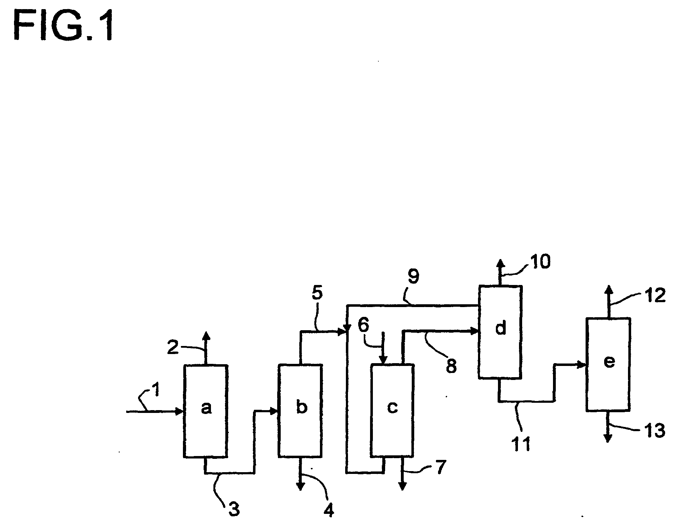

Unter Bezugnahme auf Figur 1 wird der aufzutrennende Reaktionsaustrag der Aminherstellung als Zustrom 1 einer Leichtsiederabtrennungkolonne a zugeführt. Leichtsieder sind beispielsweise unumgesetztes Edukt-Amin. Die Leichtsiederabtrennungskolonne wird im allgemeinen bei einem Druck von 1 bis 40 bar absolut, bevorzugt von 10 bis 30 bar absolut, und einer Temperatur von im allgemeinen 20 - 300°C, bevorzugt von 30 bis 250 °C betrieben. Die Zahl der theoretischen Stufen beträgt im allgemeinen 3 bis 80, bevorzugt 10 bis 30.

Am Kopf der Leichtsiederkolonne fällt ein Großteil der Leichtsieder als Kopfabzugsstrom 2 an, der in die Aminsynthese zurückgeführt werden kann. Der Sumpfabzugsstrom 3 wird der Schwersiederabtrennungkolonne b mit im allgemeinen 3 bis 80, bevorzugt 10 bis 30 theoretischen Stufen zugeführt und bei einem Druck von im allgemeinen 0,15 bis 40 bar absolut, bevorzugt 1 bis 5 bar absolut, und einer Temperatur von im allgemeinen -20 bis 300 °C, bevorzugt 30 bis 250 °C destilliert. Als Sumpfabzugsstrom 4 fallen Schwersieder an, die aus dem Verfahren ausgeschleust werden. Schwersieder sind beispielsweise Nebenprodukte mit höherem Molekulargewicht als die gewünschten Produkt-Amine. Als Kopfabzugsstrom 5 fällt ein Amin/Wasser-Azeotrop an, das noch Spuren von Leichtsiedern und Schwersiedern enthält. Durch die Schwersiederabtrennung kommt es nicht zu einer Mulmbildung im nachgeschalteten Extraktor c.

Der Kopfabzugsstrom 5 wird mit dem Seitenabzugstrom 9 der Azeotropabtrennungskolonne d vereinigt und dem Extraktor c zugeführt. Der Extraktor c kann ein- oder mehrstufig ausgebildet sein. Ein einstufiger Extraktor c ist beispielsweise ein einzelner Mixer-Settler-Extraktor. Mehrstufige Extraktoren c sind beispielsweise Extraktionskolonnen oder Extraktorkaskaden. Als Extraktionskolonnen eignen sich beispielsweise Füllkörper-, Siebboden-, Kaskaden-, Pulsations-, Rotations- und Zentrifugalkolonnen. Eine Extraktorkaskade sind beispielsweise mehrere hintereinander geschaltete Mixer-Settler-Extraktoren, die auch platzsparend als Turmextraktor oder Kastenextraktor ausgeführt sein können. Vorzugsweise ist der Extraktor c mehrstufig, besonders bevorzugt eine Gegenstrom-Extraktionskolonne mit im allgemeinen 1 bis 25, bevorzugt 4 bis 10 theoretischen Trennstufen. Diese wird im allgemeinen bei einem Druck betrieben, bei dem alle Komponenten des Extraktionsgemischs oberhalb ihres Siedepunktes vorliegen. Die Temperatur wird so gewählt, dass keine der Komponenten des Extraktionsgemischs oberhalb ihres Siedepunktes vorliegt, und sich ferner eine Viskosität der beiden Phasen einstellt, bei der eine Dispergierung der beiden Phasen problemlos möglich ist. Die Temperatur beträgt im allgemeinen 5 bis 200 °C, bevorzugt 20 bis 70 °C, beispielsweise 40 bis 50 °C. Natronlauge wird als Zustrom 6 zugegeben. Im allgemeinen beträgt die Konzentration der Natronlauge 1 bis 75 Gew.-%, bevorzugt 25 bis 50 Gew.-%. Nach Phasentrennung wird die wässrige, Natronlauge enthaltende Phase als Abstrom 7 aus dem Verfahren ausgeschleust.

Die wässrig-organische, Amin enthaltende Phase wird als Strom 8 der Azeotropabtrennungkolonne d zugeführt. Die Azeotropabtrennungskolonne weist im allgemeinen 3 bis 80, vorzugsweise 10 bis 30 theoretische Stufen auf und wird im allgemeinen bei einem Druck von 1 bis 40 bar, bevorzugt 2 bis 8 bar und einer Temperatur von -20 bis 300 °C, bevorzugt 50 bis 120 °C betrieben. Im Verstärkungsteil dieser Kolonne wird als Seitenabzugsstrom 9 ein Amin/Wasser-Azeotrop erhalten und mit dem Kopfabzugsstrom 5 der Schwersiederabtrennung vereinigt. Als Kopfabzugsstrom 10 werden weitere Leichtsieder erhalten. Als Sumpfabzugsstrom 11 wird wasserfreies Amin erhalten, das noch Spuren von Schwersiedern enthalten kann. Der Sumpfabzugsstrom 11 kann in der Reindestillationskolonne e weiter aufdestilliert werden, wobei reines Amin als Kopfabzugsstrom 12 und weitere Schwersieder als Sumpfabzugsstrom 13 erhalten werden.

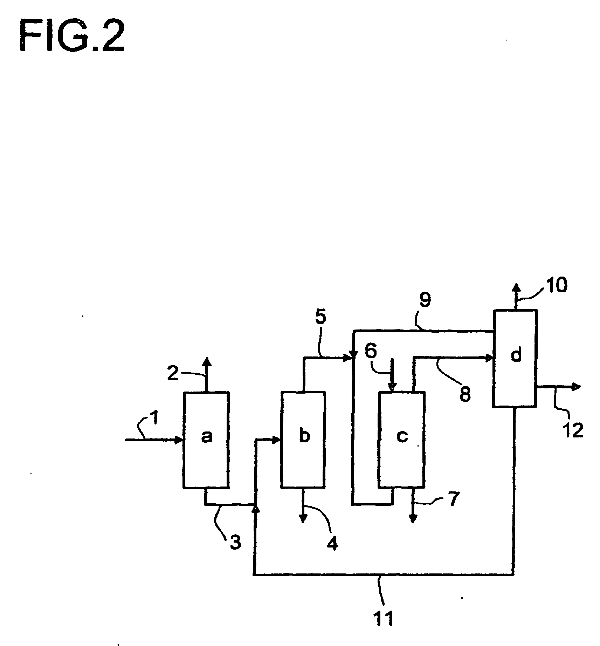

Unter Bezugnahme auf Figur 2 wird eine Variante des oben beschriebenen Verfahrens erläutert. Dabei wird der Azeotropabtrenungskolonne wasserfreies Amin nicht als Sumpfabzugsstrom, sondern als Seitenabzugsstrom 12 entnommen. Als Sumpfabzugsstrom 11 wird ein Amin mit erhöhtem Gehalt an Schwersiedern entnommen, mit dem Sumpfabzugsstrom 3 der Leichsiederabtrennung vereinigt und in die Schwersiederabtrennung zurückgeführt. Vorzugsweise beträgt der Sumpfabzugsstrom 11 0,1 bis 20 Gew.-% der Summe der Ströme 11 und 12.

Der Seitenabzugsstrom 11 kann in einer nachgeschalteten Reindestillationskolonne weiter aufgetrennt werden. Dies ist zur Gewinnung reiner Amine erforderlich, wenn im Ausgangsgemisch zwei oder mehr Amine vorliegen, die mit Wasser Azeotrope mit stark ähnlichen Siedepunkten bilden. Beispiele für solche Gemische sind N-Methylmorpholin/N-Ethylmorpholin/Wasser, Pyrollidin/N-Methylpyrollidin/Wasser und Piperidin/N-Methylpiperidin/Wasser.

Die nach dem erfindungsgemäßen Verfahren aufzutrennenden Ausgangsgemische können in ihrer Zusammensetzung stark variieren und enthalten im allgemeinen pro mol Produkt-Amin 0 bis 9 mol, bevorzugt 0 bis 3 mol Leichtsieder, 1 bis 10 mol, bevorzugt 1 bis 4 mol Wasser und, bezogen auf die Summe aller Komponenten des Ausgangsgemischs, 2 bis 20 Gew.-% Schwersieder.

Beispiele für nach dem erfindungsgemäßen Verfahren aufzutrennende Ausgangsgemische sind:
- die bei der Herstellung von Dimethylpropylamin aus Dimethylamin und Propionaldehyd in Gegenwart von Wasserstoff oder aus Dimethylamin und Propanol erhaltenen Produktgemische. Diese können als Leichtsieder unwngesetztes Dimethylamin sowie aus diesem durch Disproportionierung gebildetes Monomethylamin und Trimethylamin enthalten. Wird bei der Herstellung von Propionaldehyd ausgegangen, kann das Ausgangsgemisch die durch Reaktion von 2 Molekülen Propionaldehyd und nachfolgende Aminierung und/oder Hydrierung gebildeten Verbindungen 2-Methylpent-2-enaldehyd, 2-Methylvaleraldehyd, Dimethyl(2-methylpent-2-enyl)amin und Dimethyl(2-methylpentyl)amin als Schwersieder enthalten.
- das bei der Herstellung von Piperidin aus 1,5-Pentandiol und Ammoniak erhaltene Produktgemisch. Dieses kann als Leichtsieder unumgesetzten Ammoniak enthalten. Als Schwersieder können 2-Methylpentandiol, Dipiperidinylpentan und 2-Methylpiperidin enthalten sein.
- das bei der Herstellung von N-Methylpiperidin aus 1,5-Pentandiol und Methylamin anfallende Produktgemisch. Als Leichtsieder können alle oben genannten Methylamine enthalten sein. Schwersieder sind beispielsweise die Reaktionsprodukte von 1,5-Pentandiol mit einem oder zwei Molekülen Dimethylamin.
- das bei der Herstellung von Morpholin aus Diethylenglykol und Ammoniak anfallende Produktgemisch. Leichtsieder ist Ammoniak, Schwersieder ist beispielsweise Dimorpholinodiglykol.
- das bei der Herstellung von N-Methylmorpholin aus Diethylenglykol und Methylamin anfallende Produktgemisch. Als Leichtsieder können alle oben genannten Methylamine enthalten sein. Schwersieder sind beispielsweise die Reaktionsprodukte von Diethylenglykol mit einem oder zwei Molekülen Dimethylamin.
- das bei der Herstellung von Pyrollidin aus 1,4-Butandiol und Ammoniak erhaltene Produktgemisch. Leichsieder ist Ammoniak, Schwersieder beispielsweise die durch weitere Umsetzung von gebildetem Pyrrolidin mit unumgesetztern 1,4-Butandiol gebildeten Produkte.
- das bei der Herstellung von N-Methylpyrollidin aus 1,4-Butandiol und Methylamin erhaltene Produktgemisch. Als Leichtsieder können alle oben genannten Methylamine enthalten sein. Schwersieder sind beispielsweise die Reaktionsprodukte von 1,4-Butandiol mit einem oder zwei Molekülen Dimethylamin.

Die Erfindung wird durch das nachstehende Beispiel näher erläutert

### Beispiel

Das bei der Synthese von Piperidin aus Ammoniak und Pentandiol erhaltene Produktgemisch wird nach dem Verfahren gemäß Figur 1 aufbereitet. Der Massenstrom und die Zusammensetzung des Ausgangsgemischs (Strom 1) sowie der anderen bei der Aufarbeitung auftretenden Ströme sind in der nachfolgenden Tabelle angegeben. Das Gemisch wird bei 21 bar in einer Leichtsiederabtrennungskolonne mit 22 theoretischen Böden destilliert. Am Kopf der Kolonne wird Ammoniak abgezogen (Strom 2). Die Sumpftemperatur beträgt 204 °C. Die Kopftemperatur beträgt 46 °C. Der Sumpfaustrag der Leichtsiederkolonne (Strom 3) wird anschließend in einer Schwersiederabtrennungskolonne mit 25 theoretischen Böden bei 1 bar aufdestilliert. Am Sumpf wird ein Strom, der hauptsächlich Dipiperidinylpentan, 2-Methylpiperidin und 2-Methylpentandiol enthält, abgezogen (Strom 4). Der Kopfabzug (Strom 5) enthält Wasser, Piperidin und 2-Methylpiperidin. Die Sumpftemperatur beträgt 176 °C. Die Kopftemperatur beträgt 95 °C. Der Kopfabzugsstrom der Schwersiederabtrennungskolonne wird mit dem Seitenabzugsstrom (Strom 9) der Azeotropdestillationskolonne vereinigt und der bei Normaldruck arbeitenden Extraktionskolonne mit 10 theoretischen Trennstufen zugeführt. Die Extraktion wird bei Normaldruck mit 50 gew.-%-iger Natronlauge durchgeführt, die am Kopf der Extraktionskolonne zugegeben wird. Die Sumpftemperatur beträgt 55 °C. Die Kopftemperatur beträgt 39 °C. Als organische Phase wird am Kopf der Extraktionskolonne ein wasserarmes Gemisch mit einem Restwassergehalt von 2 Massenprozent von Piperidin und 2-Methylpiperidin abgezogen (Strom 8), das der Azeotropdestillationskolonne mit 18 theoretischen Böden zugeführt wird. Diese arbeitet bei Normaldruck. Die Sumpftemperatur beträgt 113 °C. Die Kopftemperatur beträgt 95 °C. Am dritten Boden von oben werden im Seitenabzug das Azeotrop Wasser/Piperidin und Spuren von 2-Methylpiperidin (Strom 9) abgezogen und mit dem Kopfabzugsstrom der Schwersiederabtrennung vereinigt. Der Sumpfabzugsstrom (Strom 11) der Azeotropdestillationskolonne wird der bei Normaldruck arbeitenden Reindestillationskolonne zugeführt. Am Kopf der Reindestillationskolonne fällt der Produktstrom an, dessen Hauptkomponente Piperidin ist (Strom 12). Am Sumpf wird ein Strom (Strom 13) abgetrennt, der hauptsächlich 2-Methylpiperidin enthält. Die Sumpftemperatur beträgt 123 °C. Die Kopftemperatur beträgt 109 °C. Das Verfahren wurde über einen Zeitraum von 40 Tagen ohne Feststoffbildung in der Extraktionskolonne und ohne Aufpegelung von Leichtsiedern durchgeführt.

**Tabelle**

| **Strom *** | **1** | **2** | **3** | **4** | **5** | **8** | **9** | **10** | **11** | **12** | **13** |
|---|---|---|---|---|---|---|---|---|---|---|---|
| **Wasser** | 1,136 | 0,000 | 1,136 | 0,000 | 1,136 | 0,054 | 0,050 | 0,004 | 0,001 | 0,001 | 0,000 |
| **NH3** | 2,755 | 2,755 | 0,000 | 0,000 | 0,000 | 0,000 | 0,000 | 0,000 | 0,000 | 0,000 | 0,000 |
| **Piperidin** | 2,507 | 0,000 | 2,507 | 0,003 | 2,505 | 2,606 | 0,105 | 0,009 | 2,492 | 2,480 | 0,012 |
| **2-Methylpentandiol** | 0,104 | 0,000 | 0,104 | 0,104 | 0,000 | 0,000 | 0,000 | 0,000 | 0,000 | 0,000 | 0,000 |
| **2-Methylpiperidin** | 0,084 | 0,000 | 0,084 | 0,044 | 0,040 | 0,040 | 0,000 | 0,000 | 0,040 | 0,003 | 0,037 |
| **Dipiperidinylpentan** | 0,098 | 0,000 | 0,098 | 0,098 | 0,000 | 0,000 | 0,000 | 0,000 | 0,000 | 0,000 | 0,000 |
| **Gesamtmenge** | 6,683 | 2,755 | 3,028 | 0,248 | 3,680 | 2,700 | 0,155 | 0,013 | 2,532 | 2,483 | 0,049 |

| | | | | | | | | | | | |
|---|---|---|---|---|---|---|---|---|---|---|---|
| *Angaben in kg/h | | | | | | | | | | | |

## Patentansprüche

1. Verfahren zur Auftrennung eines aminhaltigen Gemischs, das ein oder mehrere Amine, Wasser, Leichtsieder und gegebenenfalls Schwersieder enthält, mit den Schritten (i) bis (v):
(i) destillative Abtrennung einer ersten Leichtsiederfraktion von dem aminhaltigen Gemisch,
(ii) gegebenenfalls destillative Abtrennung einer (ersten) Schwersiederfraktion von dem aminhaltigen Gemisch,
(üi) Extraktion des aminhaltigen Gemischs mit Natronlauge unter Gewinnung einer wässrigen, Natronlauge enthaltenden ersten Phase und einer wässrig-organischen, Amin, weitere Leichtsieder und gegebenenfalls (weitere) Schwersieder enthaltenden zweiten Phase,
(iv) Destillation der wässrig-organischen zweiten Phase unter Gewinnung von im wesentlichen wasserfreiem Amin als Sumpfabzug oder Seitenabzug im Abtriebsteil der Destillationskolonne, einem Amin/Wasser-Azeotrop als Seitenabzug im Verstärkungsteil der Kolonne und einer weiteren Leichtsiederfraktion als Kopfabzug, und Rückführung des Amin/Wasser-Azeotrops in den Extraktionsschritt (iii),
(v) gegebenenfalls weitere Aufreinigung oder Auftrennung des im wesentlichen wasserfreien Amins.

2. Verfahren nach Anspruch 1, **dadurch gekennzeichnet, dass** Schritt (ii) durchgeführt wird.

3. Verfahren nach Anspruch 2, **dadurch gekennzeichnet, dass** in Schritt (iv) im wesentlichen wasserfreies Amin als Seitenabzug im Abtriebsteil der Kolonne gewonnen wird und weitere Schwersieder enthaltendes Amin als Sumpfabzug gewonnen wird und dieser Sumpfabzug in Schritt (ii) zurückgeführt wird.

4. Verfahren nach einem der Ansprüche 1 bis 3, **dadurch gekennzeichnet, dass** der Extraktionsschritt (üi) mehrstufig als Gegenstromextraktion in einer Extraktionskolonne mit 1 bis 25 theoretischen Trennstufen durchgeführt wird.

5. Verfahren nach einem der Ansprüche 1 bis 4, **dadurch gekennzeichnet, dass** der Destillationsschritt (iv) in einer Destillationskolonne mit 3 bis 80 Stufen bei einem Druck von 1 bis 40 bar und einer Temperatur von -20 bis 300 °C durchgeführt wird.

6. Verfahren nach einem der Ansprüche 1 bis 5, **dadurch gekennzeichnet, dass** das aufzutrennende Gemisch das bei der Herstellung von Dimethylpropylamin aus Dimethylamin und Propionaldehyd in Gegenwart von Wasserstoff oder aus Dimethylamin und Propanol erhaltene Produktgemisch ist.

7. Verfahren nach einem der Ansprüche 1 bis 5, **dadurch gekennzeichnet, dass** das aufzutrennende Gemisch das bei der Herstellung von Piperidin bzw. N-Methylpiperidin aus 1,5-Pentandiol und Ammoniak bzw. Methylamin erhaltene Produktgemisch ist.

8. Verfahren nach einem der Ansprüche 1 bis 5, **dadurch gekennzeichnet, dass** das aufzutrennende Gemisch das bei der Herstellung von Morpholin bzw. N-Methylmorpholin aus Diethylenglykol und Ammoniak bzw. Methylamin anfallende Produktgemisch ist.

9. Verfahren nach einem der Ansprüche 1 bis 5, **dadurch gekennzeichnet, dass** das aufzutrennende Gemisch das bei der Herstellung von Pyrollidin bzw. N-Methylpyrollidin aus 1,4-Butandiol und Ammoniak bzw. Methylamin erhaltene Produktgemisch ist.

## Claims

1. A process for fractionating an amine-containing mixture which comprises one or more amines, water, low-boilers with or without high-boilers, having the steps (i) to (v):
(i) separating off by distillation a first low-boiler fraction from the amine-containing mixture,
(ii) possibly separating off by distillation a (first) high-boiler fraction from the amine-containing mixture,
(iii) extracting the amine-containing mixture with sodium hydroxide solution, producing an aqueous, sodium-hydroxide-comprising first phase and an aqueous-organic, amine-, further low-boiler- and possibly (further) high-boiler-comprising second phase,
(iv) distilling the aqueous-organic second phase, producing essentially anhydrous amine as bottom-phase takeoff or sidestream takeoff in the stripping part of the distillation column, an amine/water azeotrope as sidestream takeoff in the enrichment part of the column and a further low-boiler fraction as overhead takeoff, and recycling the amine/water azeotrope to the extraction step (iii),
(v) possibly further purifying or fractionating the essentially anhydrous amine.

2. The process according to claim 1, wherein step (ii) is carried out.

3. The process according to claim 2, wherein, in step (iv), essentially anhydrous amine is produced as sidestream takeoff in the stripping part of the column and further high-boiler-comprising amine is produced as bottom-phase takeoff and this bottom-phase takeoff is recycled to step (ii).

4. The process according to one of claims 1 to 3, wherein the extraction step (iii) is carried out in multiple stages as countercurrent flow extraction in an extraction column having from 1 to 25 theoretical plates.

5. The process according to one of claims 1 to 4, wherein the distillation step (iv) is carried out in a distillation column having from 3 to 80 plates at a pressure of from 1 to 40 bar and at a temperature of from -20 to 300°C.

6. The process according to one of claims 1 to 5, wherein the mixture to be separated is the product mixture produced in the preparation of dimethylpropylamine from dimethylamine and propionaldehyde in the presence of hydrogen, or from dimethylamine and propanol.

7. The process according to one of claims 1 to 5, wherein the mixture to be separated is the product mixture produced in the preparation of piperidine or N-methylpiperidine from 1,5-pentanediol and ammonia, or methylamine, respectively.

8. The process according to one of claims 1 to 5, wherein the mixture to be separated is the product mixture produced in the preparation of morpholine or N-methylmorpholine from diethylene glycol and ammonia, or methylamine, respectively.

9. The process according to one of claims 1 to 5, wherein the mixture to be separated is the product mixture produced in the preparation of pyrrolidine or N-methylpyrrolidine from 1,4-butanediol and ammonia, or methylamine, respectively.

## Revendications

1. Procédé de séparation d'un mélange aminé qui contient une ou plusieurs amines, de l'eau, des substances à bas point d'ébullition et éventuellement des substances à haut point d'ébullition, comprenant les étapes (i) à (v) :
(i) une séparation par distillation d'une première fraction à bas point d'ébullition à partir du mélange aminé,
(ii) éventuellement une séparation par distillation d'une (première) fraction à haut point d'ébullition à partir du mélange aminé,
(iii)une extraction du mélange aminé par de la lessive de soude avec obtention d'une première phase aqueuse contenant de la lessive de soude et d'une deuxième phase aqueuse-organique contenant de l'amine, d'autres substances à bas point d'ébullition et éventuellement (d'autres) substances à haut point d'ébullition,
(iv) une distillation de la deuxième phase aqueuse-organique avec obtention d'amine sensiblement anhydre comme soutirage de fond ou soutirage latéral dans la partie de rectification de la colonne de distillation, d'un azéotrope amine/eau comme soutirage latéral dans la partie de concentration de la colonne et d'une autre fraction à bas point d'ébullition comme soutirage de tête, et un recyclage de l'azéotrope amine/eau dans l'étape d'extraction (iii),
(v) éventuellement une purification ou séparation supplémentaire de l'amine sensiblement anhydre.

2. Procédé suivant la revendication 1, **caractérisé en ce que** l'étape (ii) est effectuée.

3. Procédé suivant la revendication 2, **caractérisé en ce que**, dans l'étape (iv), on obtient de l'amine sensiblement anhydre comme soutirage latéral dans la partie de rectification de la colonne, on obtient de l'amine contenant d'autres substances à haut point d'ébullition comme soutirage de fond et on recycle ce soutirage de fond dans l'étape (ii).

4. Procédé suivant l'une des revendications 1 à 3, **caractérisé en ce que** l'étape d'extraction (iii) est effectuée en plusieurs étapes sous la forme d'une extraction à contre-courant dans une colonne d'extraction comportant 1 à 25 étages séparateurs théoriques.

5. Procédé suivant l'une des revendications 1 à 4, **caractérisé en ce que** l'étape de distillation (iv) est effectuée dans une colonne de distillation comportant 3 à 80 étages à une pression de 1 à 40 bars et à une température de -20 à 300°C.

6. Procédé suivant l'une des revendications 1 à 5, **caractérisé en ce que** le mélange à séparer est le mélange produit obtenu au cours de la préparation de diméthylpropylamine à partir de diméthylamine et d'aldéhyde propionique en présence d'hydrogène ou à partir de diméthylamine et de propanol.

7. Procédé suivant l'une des revendications 1 à 5, **caractérisé en ce que** le mélange à séparer est le mélange produit obtenu lors de la préparation de pipéridine ou respectivement de N-méthylpipéridine à partir de 1,5-pentanediol et d'ammoniac ou respectivement de méthylamine.

8. Procédé suivant l'une des revendications 1 à 5, **caractérisé** à en ce que le mélange à séparer est le mélange produit obtenu lors de la préparation de morpholine ou respectivement de N-méthylmorpholine à partir de diéthylèneglycol et d'ammoniac ou respectivement de méthylamine.

9. Procédé suivant l'une des revendications 1 à 5, **caractérisé en ce que** le mélange à séparer est le mélange produit obtenu lors de la préparation de pyrrolidine ou de N-méthylpyrrolidine à partir de 1,4-butanediol et d'ammoniac ou respectivement de méthylamine.
